# EUROPÄISCHE PATENTSCHRIFT

(11) **EP 1 560 557 B1**
(45) Veröffentlichungstag und Bekanntmachung des Hinweises auf die Patenterteilung: **01.07.2009**
(21) Anmeldenummer: 03740144.5
(22) Anmeldetag: 22.05.2003
(51) Int. Cl.: A61K 6/08, A61K 6/083

(54) **KOMPOSITMATERIAL UND VERWENDUNG EINES KOMPOSITMATERIALS**
COMPOSITE MATERIAL AND USE OF A COMPOSITE MATERIAL
MATERIAU COMPOSITE ET UTILISATION D'UN MATERIAU COMPOSITE

(30) Priorität: 13.11.2002 DE 10253481
(43) Veröffentlichungstag der Anmeldung: 10.08.2005
(73) Patentinhaber: VOCO GmbH, 27472 Cuxhaven (DE)
(72) Erfinder: MALETZ, Reinhard, 27472 Cuxhaven (DE); KRUSCHEL, Stefan, 27478 Cuxhaven (DE); PLAUMANN, Manfred, Thomas, 27476 Cuxhaven (DE)
(74) Vertreter: Hauck Patent- und Rechtsanwälte
(86) Internationale Anmeldenummer: PCT/EP2003/005338
(87) Internationale Veröffentlichungsnummer: WO 2004/043409

(56) Entgegenhaltungen:
- EP-A- 0 894 488
- WO-A-00/25729
- DE-A- 19 615 763
- US-A- 4 839 215

## Beschreibung

Die vorliegende Erfindung betrifft ein Kompositmaterial mit einem speziellen Füllstoff sowie die Verwendung eines Kompositmaterials mit einem speziellen Füllstoff für dentale Zwecke.

### Stand der Technik

Kompositmaterialien sind Verbundwerkstoffe aus einem Kunststoff und anorganischen Füllkörpern. Herkömmlicherweise bestehen sie somit grundsätzlich aus drei verschiedenen Bausteinen: einer polymerisierbaren organischen Matrix, Füllstoffpartikeln und einem Mittel, das den Verbund zwischen dem Polymer und den Füllstoffteilchen sicherstellt. Dentale, restaurative Materialien stellen eine spezielle Form der Kompositmaterialien dar, weil sie aufgrund ihrer extremen physikalischen und chemischen Belastung im überaus feindlichen Milieu des Mundes den höchsten Anforderungen ausgesetzt sind. Aufgrund ihres umfassenden Anforderungsprofils dienen diese Materialien oft als Basis zur Entwicklung nicht dentaler Komposite, bzw. als Modell zum Einsatz im nicht-dentalen Bereich.

Dentale, restaurative Kompositmaterialien werden seit über 40 Jahren für die Füllungs-, Unterfüllungs- und Befestigungstherapie, als Stumpfaufbau-, K & B- (Kronen und Brücken-), Prothesen- und Unterfütterungsmaterial, als gefüllte Adhesive, die eine Haftung an Zahnhartsubstanz, Kunststoffen, Keramiken oder Metall bewirken, sowie als Zahnversiegelungsmassen eingesetzt. Komposite härten nach Einbringen in die Kavität chemisch oder unter Zufuhr externer Energie in einer Polymerisationsreaktion aus.

Die organische polymerisationsfähige Komponente des dentalen Kompositmaterials wird in der Regel in einer radikalischen Reaktion vernetzt und enthält entsprechend ethylenisch ungesättigte, funktionelle Gruppen. Die Monomere und Oligomere umfassen die Mono-, Di- und/oder Polyacrylate und/oder Methacrylate, wie beispielsweise das Diglycidylmethacrylat des Bisphenol A ("Bis-GMA", 2,2-bis[4(2-hydroxy-3-methacryloxypropyloxy)-phenyl]propan) und das Diurethandi(meth)acrylat aus 2,2,4-Trimethylhexamethylendiisocyanat und 2-Hydroxyethyl(meth)acrylat (UDMA). Wird von Methacrylaten gesprochen, so sind auch immer die analogen Acrylate gemeint. Kommerziell erhältliche Standardmischungen enthalten Bis-GMA, UDMA sowie Triethylenglykoldimethacrylat zur Absenkung der Viskosität.

Um die Harzmischung radikalisch härten zu können, wird der Masse ein Initiatorsystem beigegeben, das die radikalische Polymerisation nach Bestrahlung und/oder dem Ablauf einer Redoxreaktion auslöst. Ein typisches System, das die radikalische Polymerisation der Methacrylate startet, besteht aus einem Photoinitiator (Keton) und einem Beschleuniger (Amin). Als Keton wird typischerweise Campherchinon, als Amin die para N,N-Dimethylaminobenzoesäure eingesetzt. Weitere photoaktive Bestandteile können der Mischung zugesetzt werden. Wird die Zusammensetzung mit einer geeigneten Stahlungsquelle bei 460 nm belichtet, so vernetzt das Kompositmaterial photochemisch. Alternativ kann das Material auch chemisch vernetzt werden. Hierzu wird die Kombination Peroxid/tertiäres Amin als Redoxsystem verwendet. Beide Komponenten müssen voneinander getrennt in einem 2-komponentigen System aufbewahrt werden. Nach dem Mischen beider Komponenten werden freie Radikale generiert und die radikalische Polymerisation der Acrylate härtet das Kompositmaterial aus. Da bei dieser Art der Härtung keine externen Hilfsmittel gebraucht werden, nennt man diese Systeme auch selbsthärtend.

Kompositzusammensetzungen können also entweder selbsthärtend oder photochemisch härtend ausgelegt sein (mono-cure). Ferner kann man Kompositzusammensetzungen formulieren, die eine Kombination aus selbsthärtenden und photochemisch härtenden Systemen darstellen (dual-cure). Wird dem einen Teil des "dual-cure" Kompositsystems noch Polyacrylsäure beziehungsweise ein Derivat der Polyacrylsäure beigefügt und ist im anderen Teil ein basisches Glas zugegen, so härtet dieses System unter geeigneten Bedingungen neben einem chemischen und photochemischen Mechanismus auch in einer Säure-Base Reaktion aus (triple cure).

Die anorganischen Füllkörper des dentalen Kompositmaterials bestehen im Allgemeinen aus Quarz, Borsilikatglas, Lithiumaluminiumsilikat, Bariumaluminiumsilikat, StrontiumBariumglas, Zinkglas, Zirkoniumsilikat, pyrogener oder kolloidaler Kieselsäure.

Der Verbund der anorganischen Füllkörper mit der organischen Harzmatrix wird in der Regel durch den Einsatz von Kupplungsreagentien oder Haflvermittlern sichergestellt. Dies ist wesentlich für die spätere Eignung der Kompositmasse als Dentalmaterial. Hierbei wird der Füllstoff, meistens in Gegenwart schwacher Säuren, mit einem Silan behandelt, bevor er mit der flüssigen Harzkomponente vermengt wird. Das Verfahren zur Präparation silanisierter Füllstoffoberflächen besteht darin, eine Ethanol/Wasser Mischung (meistens 95/5 Vol%) zunächst mit Essigsäure auf einen pH-Wert von 4.5 - 5.5 einzustellen. Das Silan wird sodann in einer solchen Menge zugegeben, daß eine Lösungskonzentration von ca. 2% resultiert. Innerhalb von 5 Minuten sind die Alkoxysilylgruppen hydrolysiert und die Siloxanbildung setzt ein. Nun wird der zu behandelnde Füllstoff unter fortgesetztem Rühren der Lösung beigegeben. Innerhalb weniger Minuten wird das Silan vom Füllstoff adsorbiert und die Oberfläche der Füllkörper vom Haftvermittler beladen. Die Lösung wird abdekantiert und die Partikel werden zweimal mit Ethanol gewaschen.

Abschließend werden die restlichen Silanolfunktionen für wenige Minuten bei 110°C und 24 Stunden bei Raumtemperatur kondensiert.

Das Silan agiert als oberflächenaktiver Stoff, der die Oberfläche des Füllstoffs mit der Harzmatrix kompatibilisiert und für einen festen Verbund zwischen dem organischen und dem anorganischen Material sorgt. Als besonders geeignetes Silan zum Aufbau eines Verbundes zwischen der anorganischen und der organischen Phase hat sich unter anderem das 3-Methacryloyloxypropyltrimethoxysilan erwiesen. Ein Teil der hydrolysierten Alkoxysilylgruppen des Silans reagieren direkt mit den Hydroxylgruppen auf der mineralischen Oberfläche des Füllstoffs während der andere Teil untereinander kondensiert und so eine miteinander zusammenhängende Schicht des Kupplungsreagenzes auf der Füllstoffoberfläche ergibt. Im Verlauf der später stattfindenden radikalischen Polymerisation der dentalen Kompositmasse werden dann die Methacryloyloxypropylfunktionen der an der Füllstoffoberfläche haftenden durchgängigen Schicht des Silans in die organische Harzphase mit einpolymerisiert und bilden so einen dauerhaften Verbund zwischen den hydrophilen Füllstoffen und der hydrophoben Harzmatrix.

Das Eigenschaftsprofil des resultierenden dentalen Verbundwerkstoffes wird in erster Linie von der anorganischen Phase bestimmt. Während Youngs Modul (E-Modul) für ein nichtgefülltes Harzsystem auf Bis-GMA-Basis 2.8 GPa beträgt, weist der Zahnschmelz einen Wert von 83 GPa und das Dentin einen Wert von 19 GPa auf. Durch Zugabe eines konventionellen, silylierten Füllstoffs zum Bis-GMA-Harz kann der Wert von 2.8 GPa deutlich verbessert werden. Wird der Füllstoff im Volumenverhältnis von 1 zu 1.25 dem Harz beigegeben, so kann Youngs Modul auf einen Wert von 10 GPa angehoben werden. Für ein Verhältnis 1 zu 1 kann ein Wert von 15 GPa erreicht werden.

Füllstofftyp, Menge und Partikelverteilung bestimmen für eine gegebene Harzzusammensetzung die mechanischen, ästhetischen und rheologischen Kennzeichen des dentalen Kompositformstoffs wie Oberflächenhärte, Abriebbeständigkeit, Verschleißfestigkeit, Druckfestigkeit, Zugfestigkeit, Polymerisationsschrumpfung, Frakturresitenz und Wärmewechselbeständigkeit sowie Polierbarkeit, Glanz, Opazität, Transluzenz und Farbstabilität sowie Fließverhalten, Standfestigkeit und Modellierbarkeit. Als Faustregel gilt: Je höher die Beladung des flüssigen Harzes mit silanisiertem Füllstoff, umso besser die mechanischen, physikalischen und chemischen Eigenschaften des ausgehärteten Formstoffs.

Vor dem Hintergrund der überragenden Bedeutung der anorganischen Phase für die Eigenschaften dentaler Kompositmaterialien läßt sich auch die traditionelle Einteilung dentaler Kompositwerkstoffe in drei verschiedenen Grundklassen verstehen.

Ein makrogefülltes Kompositmaterial ist eine mit relativ großen Partikeln (1-100 µm), hochgefüllte (bis 87 Gew.%) Zusammensetzung. Während als Füllstoff früher Glaspulver mit mittleren Korngrößen von 30 - 50 µm diente, ist der Füllstoff heute meistens gemahlener Quarz oder auch eine Glaskeramik mit einer mittleren Teilchengröße von 8 - 12 µm. Makrogefüllte Komposite weisen die beste Verschleißfestigkeit auf, lassen sich jedoch aufgrund der Partikelgröße außerordentlich schlecht hochglanzpolieren. Während der Politur brechen die voluminösen Füllstoffpartikel aus der Füllung aus, es bleiben kleine Löcher zurück und die ausgebrochenen Füllstoffsplitter üben einen Schmirgeleffekt auf den restlichen Formstoff aus, so daß sich makrogefüllte Komposite nicht hochglanzpolieren lassen und ein grundsätzliches ästhetisches Defizit aufweisen.

Um der Forderung nach verbesserter Ästhetik nachzukommen, wurde die Gruppe der mikrogefüllten dentalen Kompositmaterialien entwickelt. Charakteristisches Kennzeichen dieser Gruppe ist die außergewöhnlich kleine Partikelgröße der Kompositfüllstoffe, die in erster Linie aus amorpher Kieselsäure bestehen und eine mittlere Teilchengröße von ca. 0.04 µm aufweisen. Diese kleine Partikelgröße bedingt eine extrem große Teilchenoberfläche, die ihrerseits infolge intensiver Wechselwirkungskräfte zwischen den Partikeloberflächen der Füllstoffbeladung des Kompositmaterials eine frühe Grenze setzt. In der Regel können mikrogefüllte Kompositmaterialien nicht über 50 Gew% mit Füllstoff versetzt werden, da das Material aufgrund zu hoher Viskosität dann nicht mehr verarbeitbar ist. Diese Kompositklasse ist hochglanzpolierbar, zeigt ausgezeichnete refraktive Eigenschaften und erfüllt alle Kriterien eines äußerst ästhetisch wirkenden dentalen Werkstoffes. Bedingt durch den geringen Füllstoffgehalt zeigen mikrogefüllte Werkstoffe verglichen mit den makrogefüllten dentalen Kompositen jedoch stark reduzierte mechanische Eigenschaften wie Abrieb, Zugfestigkeit, zu hohen Schrumpf, etc.

Es wurde vielfach, bisher jedoch vergeblich, versucht, den Füllstoffgehalt beispielsweise durch Einbau der pyrogene Kieselsäure in vorpolymerisierte Harzpartikel (25 µm), agglomerierte oder gesinterte Teilchen zu erhöhen und so die Festigkeitswerte zu erhöhen.

Durch den Versuch, die Hochglanzpolierbarkeit der mikrogefüllten Verbundwerkstoffe mit den guten mechanischen Eigenschaften der makrogefüllten Komposite zu kombinieren, entwickelte man die Klasse der sogenannten Hybridkomposite. Hierbei ist der eingesetzte Füllstoff eine Mischung aus konventionellem Glas mit einer Partikelgröße von 0.6 - 1.5 µm sowie aus nanoskaligen Teilchen von 0.01 - 0.05 µm. In der Regel beträgt der mengenmäßige Anteil der nanoskaligen Kieselsäureteilchen 7 - 15 Gew.%. Der Gesamtfüllstoffgehalt kann bis zu 80 Gew-% betragen. Aufgrund der großen Variation in den Partikelgrößen kann so eine äußerst kompakte Packungsdichte der Füllstoffteilchen erzielt werden, wobei kleinere Partikel in den Zwischenräumen der größeren Partikel zu liegen kommen.

Ein Beispiel für die Zusammensetzung eines mikrogefüllten Systems wird in der DE 2403211 beschrieben. Hybridmaterialien sind aus den Patentschriften DE 2405578, DE 3403040 und EP 382033 bekannt.

Die US 4,839,215 A beschreibt hohlzylindrische Füllstoffteilchen und dentale Kompositmaterialien mit diesen Füllstoffen, wobei die Füllstoffe über das Bindemittel mechanisch verkettet sind und somit die mechanischen Eigenschaften der auspolymerisierten Kompositmaterialien verbessern. Die bekannten Füllstoffteilchen haben eine kreiszylindrische Form. Die zentrale Öffnung der Teilchen liegt zwischen 500 und 1.000 µm und bevorzugt zwischen 150 bis 400µm, am bevorzugsten zwischen 225 und 300 µm. Der Außendurchmesser der Teilchen liegt unter 3 µm, bevorzugt zwischen 425 und 200 µm, mehr bevorzugt zwischen 500 und 1.000 µm und am bevorzugsten um 925 µm. Bevorzugt werden die Keramikteilchen durch Pressen hergestellt. Als weitere Herstellungsmethoden sind die Extrusion, das Gießen, isostatisches Pressen, Heißpressen, Spritzgießen und Ablagern auf einem Formkörper angegeben. Die groben Füllstoffteilchen ermöglichen nur Kompositmaterialien, die geringe Festigkeiten, geringe Härten, geringe Abrasionsresistenzen und schlechte Polierbarkeiten aufweisen.

### Aufgabe der Erfindung

Obwohl moderne Kompositfüllungen, bedingt durch die werkstoffkundlichen Verbesserungen, nun auch im Seitenzahnbereich einen festen Platz in der Behandlungspalette der Zahnärzte einnehmen, weisen diese Systeme dennoch einige grundsätzliche Schwächen auf, die in erster Linie mit dem "Verbund" zwischen der organischen Harzmatrix und den anorganischen Füllstoffoberflächen zusammenhängen. Die Silan-Kupplungsmittel bilden "Siloxanbindungen" mit Mineralien. Diese Bindungen, die für den Verbund zwischen den beiden Phasen sorgen, sind, wie jede Bindung zwischen einem organischen Polymer und einer hydrophilen, mineralischen Feststoffoberfläche, hydrolysierbar. Eine Hydrolyse der Siloxanbindung bewirkt jedoch hydrolytischen Abbau im Polymer, verstärkte Rißbildung entlang der Grenzflächenregion Feststoff/Harz, Wasserabsorption, weichmachende Effekte im Polymer, Quellung des Komposits, verminderte Verschleißfestigkeit, Abriebfestigkeit und Farbstabilität durch Herausbrechen der Füllstoffe. Letztendlich wird der Verbund beider Phasen gelöst.

Der Vorteil der Silane gegenüber anderen Haftvermittlern liegt in ihrer Eigenschaft, sich bezüglich der hydrolytischen Bindungsöffnung reversibel zu verhalten. Das thermodynamische Gleichgewicht liegt weit auf der Seite der Siloxan-Bindungsbildung. Obwohl demnach die Gleichgewichtsmenge der Wassermoleküle an der Grenzschicht Polymer/Feststoff wichtiger als die Diffusionsgeschwindigkeit des Wassers in das Polymer ist, wird dennoch in den Werkstoff eindringendes Wasser den hydrolytischen Abbauprozess in Gang setzen. Wasser dringt selbst bei stark hydrophoben Harzen durch Diffusion bis zur Grenzfläche Polymer/Feststoff vor. Ist die Grenzschicht einmal angegriffen, wird sich das Wasser dort in Form von Clustern anlagern, den Verbund der organischen Phase zur anorganischen Phase lockern und durch osmotischen Druck das Gefüge des Komposits aufreißen.

Zur Verbesserung des Verbundes zwischen Füllstoff und Polymer-Matrix wurde die Möglichkeit in Betracht gezogen, zusätzlich zur chemischen Haftung eine physikalische Haftung aufzubauen. In US 4215033 wird durch Ätzen eines Glases ein semiporöser Füllstoff geschaffen. Mikroporöse Füllstoffe für den Einsatz als Dentalmaterialien sind aus den Schriften US 4217264, EP 4868, EP 172513, DE 198 46 556 und DE 19615763 bekannt. Bei der physikalischen Haftung dringt Harz in die Poren des Füllstoffs ein und verankert so nach der Polymerisation die organische mit der anorganischen Phase, da das ausgehärtete Harz fest in den Poren des Füllstoffs gehalten wird. Somit wird eine bessere strukturelle Integrität des Formstoffes gewährleistet.

In US 4,839,215 werden torusförmige Füllstoffteilchen und dentale Kompositmaterialien mit diesen Füllstoffen beschrieben, wobei die Füllstoffe über das Bindemittel mechanisch verbunden sind und somit die mechanischen Eigenschaften der auspolymerisierten Kompisitmaterialien verbessern. Der Torusaußendurchmesser liegt zwischen 425 und 200 Mikrometern.

Das Prinzip der physikalischen Verankerung von Füllstoff und Matrix, das in DE 196 15 763 beschrieben wird und die Verwendung poröser SiO₂ Partikel umfaßt, weist jedoch drei prinzipielle Nachteile auf. Der erste besteht in der sehr aufwendigen Herstellung der porösen Füllkörper, die einen sehr kostspieligen Phasentrennungsschritt, sowie Aufmahlungs- und Sichtungsverfahren beinhaltet. Der zweite Nachteil liegt in dem sehr geringen Porendurchmesser, der vorzugsweise 90-100 Nanometer beträgt. Um ein effektives Einfließen des Harzes in die Poren zu gewährleisten, müssen sehr niederviskose Harzgemische mit geringer Oberflächenspannung zur Anwendung kommen. Diese erhält man durch Verwendung von Dimethacrylaten mit geringem Molekulargewicht wie z.B. dem Triethylenglycoldimethacrylat (TEDMA) oder dem Hexandioldimethacrylat (HEDMA). Ein höherer Anteil dieser niedermolekularen Monomere führt beim Komposit jedoch zu einer erhöhten Schrumpfung. Alternativ kann die Viskosität der Matrix auch durch Zusatz von Monomethacrylaten wie z.B. dem Hydroxypropylmethacrylat (HPMA) oder dem Triethylenglycolmonoethylethermonomethacrylat gesenkt werden. Die Verwendung von Monomethacrylaten führt im Vergleich zu Dimethacrylaten zu einer schlechteren Vernetzung des Polymers und somit zu geringeren Biegefestigkeiten und zu einer höheren Verfärbung. Der dritte Nachteil liegt in der Einschränkung des Herstellungsverfahrens auf Siliziumdioxid-Füllstoffe, die keine Einstellung einer klinisch akzeptablen Röntgenopazität erlauben.

Die WO 00/25729 A1 offenbart ein gefülltes Dentalmaterial mit verbesserten mechanischen Eigenschaften, umfassend eine Harzmatrix und eine Füllstoffkomponente. Die Füllstoffkomponente enthält 5 bis 35 Gew.-% Keramikteilchen mit einer bestimmten Form. Die bestimmte Form ist ausgewählt aus der Gruppe bestehend aus Kugel, Ringwulsten, Multidellen, Hohlkugeln, Nuggets, Stäbchen, Fasern und Mischungen davon. Das gefüllte Dentalmaterial ist aufgrund eines mechanischen Ineinandergreifens der Keramikteilchen innerhalb der Matrix verbessert.

Die Keramikteilchen werden durch ein naßchemisches Verfahren hergestellt. Der mittlere Durchmesser der wulstförmigen Teilchen beträgt etwa 5 µm. Nach ergänzenden Angaben der Anmelderin der WO 00/25729 A1 im europäischen Prüfungsverfahren zur EP 1 124 529 B1 sind die durch das naßchemische Verfahren hergestellten Partikel mit Öffnungen versehen, in die das Matrixmaterial eindringt und mit denen es sich verzahnen kann. Das Matrixmaterial kann in eine Öffnung an der Ober- und Unterseite und in die porösen Wände der ringwulstförmigen Partikel eingreifen. Die poröse Oberfläche der Partikel ist in rasterelektronenmikroskopischen (REM) Aufnahmen sichtbar.

Die Porosität der Teilchen beruht auf der naßchemischen Synthese und der nachfolgenden Wärmebehandlung der Partikel. Die Partikel werden nämlich bei der Herstellung in einem Tiegel in einem Ofen über 24 Stunden bei einer Temperatur von etwa 600°C erhitzt. Diese Temperaturbehandlung schließt die Umwandlung von Kieselgel in Kieselglas und in einigen Fällen die Bildung der Löcher in den Teilchen ab.

Die Porosität der bekannten Füllstoffpartikel beeinträchtigt deren Kompatibialität mit dem Matrixmaterial. Folglich ist der maximale Gewichtsanteil der Füllstoffteilchen im Dentalmaterial verhältnismäßig gering. Dies beeinträchtigt die mechanischen Eigenschaften und das Schrumpfverhalten nach der Polymerisation des vorbekannten Dentalmaterials.

Trotz enormer Verbesserungen auf dem Feld der dentalen Kompositmaterialien bleibt das Problem des Phasenverbundes dennoch ungelöst, da es auch bei Einsatz poröser Füllstoffe zu der durch hydrolytische Spaltung bedingten Ablösung der Polymermatrix vom anorganischen Füllstoff kommen kann. Es ist daher die Aufgabe der Erfindung, einen Füllstoff zur Verfügung zu stellen, der einen stabilen Verbund mit der organischen Phase eingeht und eine so starke physikalische Bindung zwischen ihm und dem Bindemittel des Dentalmaterials auszubilden vermag, daß eine möglicherweise stattfindende Hydrolyse den einmal gebildeten Verbund nicht mehr zerstören kann, sowie ein diesen Füllstoff enthaltendes Kompositmaterial, das aufgrund des stabilen Verbundes zwischen den Phasen ein gegenüber dem Stand der Technik verbessertes Eigenschaftsprofil gewährleistet.

### Detaillierte Beschreibung der Erfindung

Die Aufgabe wird durch ein Kompositmaterial gemäß Anspruch 1 und durch ein dentales Kompositmaterial gemäß Anspruch 15 und durch die Verwendung eines Kompositmaterials gemäß Anspruch 16 gelöst. Vorteilhafte Ausgestaltungen der Erfindung sind in den Unteransprüchen abgegeben.

Das erfindungsgemäße Kompositmaterial mit einem polymerisierbaren organischen Bindemittel und einem Füllstoff, das durch Sprühtrocknung von Solen erhaltene Füllstoffteilchen enthält, welche die Form eines Torus und einen mittleren Außendurchmesser im Bereich von 0,5 - 100 µm aufweisen, ist **dadurch gekennzeichnet, dass** die Füllstoffteilchen mit der Form eines Torus bei einer Temperatur im Bereich von 800 bis 900 °C getempert sind.

Die Erfindung bezieht sich auf ein Kompositmaterial mit speziellem Füllstoff mit Füllstoffteilchen, die eine sphärisch ringförmige Struktur aufweisen, strukturell der Geometrie eines Torus entsprechen, bei einer Temperatur im Bereich von 800 bis 900 °C getempert sind und einen mittleren Außendurchmesser im Bereich von 0,5 bis 100 µm aufweisen. Im Gegensatz zu den porösen Füllstoffen, bei denen das Harz in den Poren der Füllstoffpartikel lediglich mechanisch verankert ist, wird hier der Extremfall verwirklicht, indem eine einzige Pore den ansonsten porenfreien Füllstoff komplett durchzieht. Dies führt dazu, daß sich die organische Phase kontinuierlich mit der anorganischen Phase verbindet und so eine unter mechanischen Gesichtspunkten besonders effektive Verkettung bildet. Die torusförmigen Füllstoffpartikel werden dabei mechanisch wie die Perlen einer Kette vom Bindemittel durchgriffen und durch die im Inneren des Torus vorliegende durchgängige Harzphase so miteinander verbunden, daß sie durch hydrolytischen Abbau nicht mehr von der Harzmatrix abgelöst werden können. Man erhält so einen Dentalwerkstoff, der aufgrund des äußerst wirksamen Verbundes zwischen organischer und anorganischer Kompositphase eine besonders ausgeprägte Abrasionsfestigkeit bei gleichzeitig hoher Biegefestigkeit aufweist, der von vergleichbaren Dentalwerkstoffen des Standes der Technik nicht erreicht werden kann. Da auch der im Laufe der Zeit einsetzende hydrolytische Abbau wegen des festen Phasenverbundes nicht mehr zu einer Phasentrennung führt, wird somit die Haltbarkeit des Dentalwerkstoffes bei voller Funktionsfähigkeit verlängert. Gleichzeitig wird auch der ästhetische Charakter der dentalen Masse erhöht. Während des Polierens werden die Füllstoffpartikel aufgrund des festen Phasenverbundes mit dem Bindemittel schichtweise abgetragen und nicht, wie bei den makrogefüllten Kompositen, als Ganzes aus der polymeren Matrix gebrochen. Dies ermöglicht eine Hochglanzpolitur.

Geeignete Bindemittel für Kompositwerkstoffe sind neben den ethylenisch ungesättigten Monomeren und Oligomeren die Epoxide, Ormocere, Ceramere, flüssigkristalline Systeme, Spiroorthoester, Oxethane, Polyurethane, Polyester, A-Silikone und C-Silikone, Polycarbonsäuren etc.

Weitere mögliche Bestandteile des Kompositwerkstoffes umfassen Farbstoffe, Pigmente, Stabilisatoren, Co-Initiatoren, Benetzungsmittel, Röntgenopaker etc.

Ferner bezieht sich die Erfindung auf Kompositmaterialien mit dem Füllstoff mit torusförmigen Füllstoffteilchen für nicht-dentale Zwecke und auf den Füllstoff mit torusförmigen Füllstoffteilchen für beliebige Zwecke.

Nachfolgend wird ein Verfahren zur Herstellung der torusförmigen Füllstoffteilchen erläutert.

Die ringförmigen, sphärischen Füllstoffe lassen sich aus amorphen, nanoskaligen SiO₂-Primärpartikeln aufbauen. Bevorzugt werden hierfür kolloidale Kieselgele als Suspension in Wasser (Kieselsole) eingesetzte. Diese Kieselgele können sowohl Ammonium, Aluminium als auch Natrium als stabilisierendes Genion enthalten. Die bevorzugte Primärpartikelgröße liegt bei 5 - 100 nm, die am meisten bevorzugte Primärpartikelgröße bei 10 - 50 nm. Gängige SiO₂-Suspensionen sind z.B. das Ludox AS40 oder das Ludox HS40 (Aldrich Chemical Company, Milwaukee, USA).

Um eine ausreichende Röntgensichtbarkeit der Dentalmaterialien zu erlangen, werden Schwermetalloxide in Kombination mit SiO₂ in die ringförmigen Füllstoffe eingebaut. Bevorzugt werden die Oxide von Schwermetallen mit einer Ordnungszahl größer 28 verwendet. Besonders bevorzugt werden die Oxide von Yttrium, Strontium, Barium, Zirkon, Wolfram, Zinn, Zink, Lanthan oder Ytterbium Oder Kombinationen hieraus eingesetzt. Die Schwermetalle können in den Herstellungsprozess in Form von Lösungen, Solen oder Partikelsuspensionen eingebracht werden. Hierbei liegt die bevorzugte Größe der Schwermetallpartikel bei 5 bis 100 nm, die besonders bevorzugte Größe bei 10 - 50 nm. Als Vorstufen für die Schwermetalloxide können wasserlöslich anorganische oder organische Salze der entsprechenden Metalle wie z.B. die Salz von aliphatischen Mono- oder Dicarbonsäuren oder auch Alkoholate eingesetzt werden. Bevorzugt verwendet man Zirkoniumacetat. Das Elementverhältnis Silizium . Schwermetall kann hierbei 0.3:1 bis 20:1 betragen. Bevorzugt liegt das Verhältnis bei 2:1 bis 8:1.

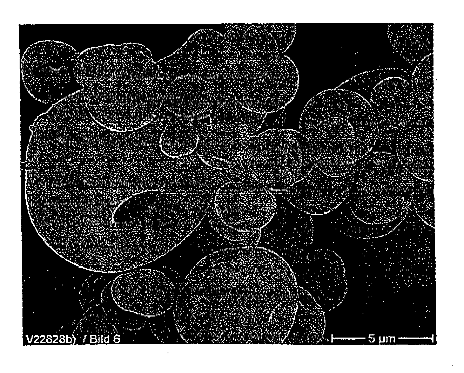

Zur Herstellung der Torus-Füllstoffe wurden entweder die reinen Kieselsole und/oder wässrige Gemische der Kieselsole und der Schwermetallsalze vom Wasser und anderen flüchtigen Bestandteilen befreit. Die bevorzugte Methode zur Herstellung von sphärischen, nicht-agglomerierten Partikeln im Mikrometerbereich stellt die Sprühtrocknung solcher Sole dar. Hierfür kam ein Sprühtrockner "Mobile Minor 2000" der Firma Niro A/S Soborg, Dänemark, zum Einsatz. Es wurden unterschiedliche Düsengeometrien (Zweistoff-, Zentrifugal- und Springbrunnendüsen), Zulufttemperaturen im Bereich 150-300° C, Feststoffkonzentrationen im Bereich 1-40 Gew.-%, Sprühdrücke im Bereich 2-5 bar und Durchflussraten im Bereich 0.2 - 2.0 Kg/h gestestet. Überraschend wurde gefunden, dass im obigen Bereich der unterschiedlichen Prozessparameter sich Partikel mit Torusform ausbildeten. Die Abbildung 1 zeigt REM-Aufnahmen von solchen SiO₂-Partikeln, die Torusstruktur aufweisen.

Es lassen sich Füllstoffpartikel mit einem Außendurchmesser im Bereich 0.5 - 100 µm, bevorzugt im Bereich 1-50 µm herstellen. Der Durchmesser der Ringöffnungen liegt im Bereich 0.2 - 20 µm, bevorzugt im Bereich 0.4 - 4.0 µm.

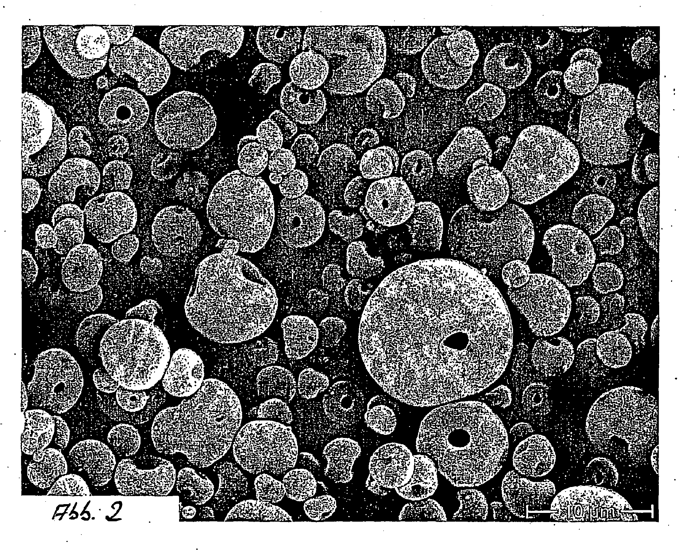

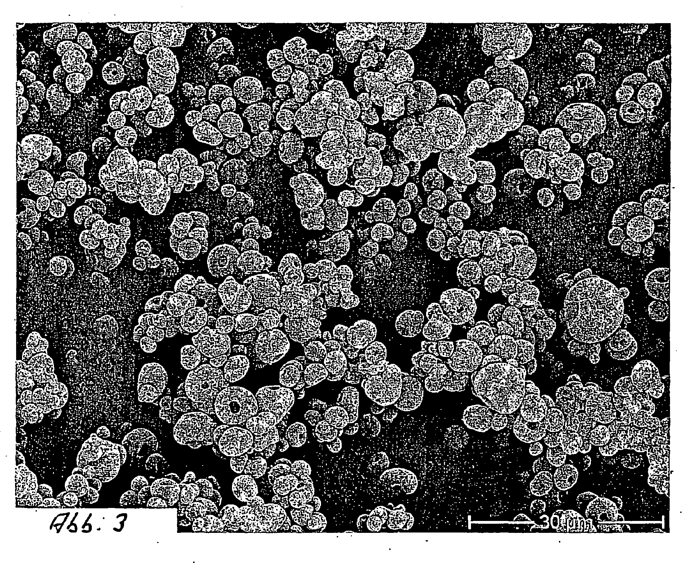

Die durch Sprühtrocknung erhaltenen Partikel zeigten eine mäßige mechanische Stabilität und ließen sich durch die Einwirkung von Scherkräften, die bei der Herstellung hochviskoser, dentaler Füllungsmaterialien auftreten, zerstören. Eine hohe mechanische Festigkeit der Partikel wurde durch Calcinieren erreicht .Das Tempern der Füllstoffe wurde bei 800-900°C durchgeführt. Nach dem Tempern wurde keine Verkleinerung der Partikel festgestellt Auch die Torusstruktur bleibt nach der thermischen Kondensation unverändert (Abb. 2). Das Tempern bei 800 - 900°C führt zu Füllstoff-Agglomeraten (Abb. 3), die jedoch durch Zuführung mechanischer Energie wie z.B. Ultraschall, unter Erhalt der Ringstruktur, wieder deagglomeriert werden können. Die erfindungsgemäßen Füllstoffe werden silanisiert, um sowohl eine zusätzlich chemische Verknüpfung der Füllstoffe mit der Matrix nach Aushärtung des Komposites zu ermöglichen, als auch eine Hydrophobierung der Partikeloberflächen zu erreichen, die das Durchfließen der Füllstoffe mit der hydrophoben Matrix erleichtert. Hierfür werden 100 g des Füllstoffs mit einer Lösung von 5 g Methacryloyaloxypropyltrimethoxysilan und 5 g Wasser in 90 g Ethanol, die mit Essigsäure auf pH-Wert von 5 eingestellt wurde, versetzt, die Lösung nach Rühren abdekantiert, der Füllstoff mit Ethanol gewaschen, isoliert und anschließend 10 Minuten bei 110°C und 24 h bei Raumtemperatur getrocknet.

### Ausführungsbeispiele

Die Eigenschaften der Torus-Füllstoffe wurden anhand experimenteller, lichthärtender dentaler Füllungskomposite untersucht. Hierfür wurden die nachfolgenden Zusammensetzungen in einem Vakuum-Planetenmischer formuliert und bei einem Unterdruck von 0.95 bar entlüftet.

Als ethylenisch ungesättigte Harzkomponenten kamen zum Einsatz: Triethylenglycoldimethacrylat (TEDMA), Bisphenol-A-diglycidylmethanacrylat (BisGMA) und Diurethandimethacrylat (UDMA). In Beispiel 5 wurde ein 40 %-iges Sol von SiO₂-Partikeln mit einer Primärpartikelgröße von 15 nm in einer Dimethacrylatmischung Bis-GMA:UDMA:TEDMA von 5:3:2 eingesetzt.

Als Füllstoffe wruden neben den erfindungsgemäßen Toruspartikeln mit einer durchschnittlichen Korngröße von 3.0 µm sphärische Siliziumdioxidpartikel mit einer durchschnittlichen Korngröße von 3.0 µm, sowie splitterförmige Barium-Aliminium-Borosilikat-Gläser mit durchschnittlichen Korngrößen von 3.0 bzw. 0.7 µm verwendet. Die Glasfüllstoffe wurden nach gleichem Verfahren wie die Toruspartikel silanisiert.

Zur Einstellung der verarbeitungsgerechten Konsistenz wurde pyrogene Kieselsäure (HDK H2000, Fa. Wacker, München) eingesetzt.

Als Initiatorsystem für die Blaulichthärtung kam Campherchinon / 4-(N, N-Dimethylamino)Benzoesäureethylester (DMAE) zum Einsatz.

Die Lagerstabilität der Materialien wurde durch Zusatz von Butylhydroxytoluol (BHT) erhöht.

Die Aushärtung der Prüfkörper zur Durchführung der werkstoffkundlichen Untersuchungen erfolgte mit dem Halogenlichtgerät Polofil Lux (VOCO GmbH, Cuxhaven) mit einer Lichtintensität von 750 mW/cm².

Zur Charakterisierung der Festigkeit der experimentellen Füllungskomposite wurde die Biegebruchfestigkeit nach ISO 4049 Pkt. 2.11 bestimmt. Die Messung der Polymerisationsschrumpfung wurde mit einem Dilatometer 30 Minuten nach Belichtung bestimmt Geräteaufbau und Methode sind in der Publikation "Curing contraction of composites and glass-ionomer cements, A.J. Feilzer, A.J. De Gee, C.L. Davidson, Journal of Prosthetic Dentistry, Vol. 59, Nr. 3, S297-300", angegeben. Die Abriebbeständigkeit der Komposite wurde mittels der Drei-Medien-Abrasion gemessen, beschrieben in "Occlusal wear simulation with the AVTA wear machine, A.J. De Gee, P. Pallav, J. Dent. Suppl. 1, 1994, 22, S21-27".

### Beispiel 1

| | |
|---|---|
| Ba-Al-Borosilikatglas 3.0 µm | 61.2 g |
| Pyrogene Kieselsäure | 4.9 g |
| Bis-GMA | 16.9 g |
| UDMA | 10.1 g |
| TEDMA | 6.7 g |
| Campherchinon | 0.07 g |
| DMABE | 0.07 g |
| BHT | 0.02 g |

### Beispiel 2

| | |
|---|---|
| Spärische SiO₂-Partikel, 3.0 µm | 61.2 g |
| Pyrogene Kieselsäure | 4.9 g |
| Bis-GMA | 16.9 g |
| UDMA | 10.1 g |
| TEDMA | 6.7 g |
| Campherchinon | 0.07 g |
| DMABE | 0.07 g |
| BHT | 0.02 |

### Beispiel 3

| | |
|---|---|
| Torus-Partikel, 3.0 µm | 61.2 g |
| Pyrogene Kieselsäure | 4.9 g |
| Bis-GMA | 16.9 g |
| UDMA | 10.1 g |
| TEDMA | 6.7 g |
| Campherchinon | 0.07 g |
| DMABE | 0.07 g |
| BHT | 0.02 g |

### Beispiel 4 :

| | |
|---|---|
| Torus-Partikel, 3.0 mm | 56.2 g |
| Ba-Al-Borosilikatglas, 0.7 mm | 10.0 g |
| Pyrogene Kieselsäure | 4.9 g |
| Bis-GMA | 14.9 g |
| UDMA | 8.1 g |
| TEDMA | 5.7 g |
| Campherchinon | 0.07 g |
| DMABE | 0.07 g |
| BHT | 0.02 g |

### Beispiel 5:

| | |
|---|---|
| Torus-Partikel | 58.5 g |
| 40%-iges SiO₂-Sol | 41.3 g |
| Campherchinon | 0.07 g |
| DMABE | 0.07 g |
| BHT | 0.02 g |

| | Biegef. (MPa) | Abrasion (µm) | Schrumpfung |
|---|---|---|---|
| (Vol%) | | | |
| Beispiel 1 | 102 | 77 | |
| | 3.85 | | |
| Beispiel 2 | 104 | 61 | |
| | 3.41 | | |
| Beispiel 3 | 135 | 44 | |
| | 3.09 | | |
| Beispiel 4 | 131 | 39 | |
| | 2.84 | | |
| Beispiel 5 | 144 | 29 | |
| | 2.49 | | |

Der Austausch der splitterförmigen Glaspartikel (Beispiel 1) durch sphärische Siliziumdioxidteilchen (Beispiel 2) führt zu einer nur geringförmigen Verbesserung der Abriebresistenz und der Polymerisationsschrumpfung.

Der Vergleich zu einer Standard-Kompositzusammensetzung (Beispiel 1) zeigt, dass der Ersatz von splitterförmigen Glasfüllstoffen durch die erfindungsgemäßen Torus-Füllstoffe bei ansonsten gleichen Rezeptur-Parametern (Beispiel 3) aufgrund der optimalen Verankerung der Füllstoffe in der ausgehärteten Matrix zu einer höheren Festigkeit führt. Diese spezielle Verankerung führt auch zu einer deutlich verbesserten Abrasionsresistenz, da die Füllstoffe nur schwerer am Stück herausgerissen werden können. Die hohe Raumerfüllung des anorganischen Feststoffs aufgrund der sphärischen Struktur und der damit verbundenen günstigen Packung der Partikel ergibt eine geringe Volumenschrumpfung.

Unter Zusatz von feinen Glasfüllstoffen zu den Toruspartikeln (Beispiel 4) lässt sich der anorganische Füllstoffanteil nochmals erhöhen, sodass das Komposit verbesserte Abrasions- und Schumpfungswerte aufweist.

Der Einsatz nanoskaliger SiO₂-Partikel (Beispiel 5) scheint zu einer optimalen Raumerfüllung der Füllkörper zu führen, die die mechanischen Eigenschaften nochmals verbessert.

## Patentansprüche

1. Kompositmaterial mit einem polymerisierbaren organischen Bindemittel und einem Füllstoff, das durch Sprühtrocknung von Solen erhaltene Füllstoffteilchen enthält, welche die Form eines Torus und einen mittleren Außendurchmesser im Bereich von 0,5 - 100 µm aufweisen, **dadurch gekennzeichnet, dass** die Füllstoffteilchen mit der Form eines Torus bei einer Temperatur im Bereich von 800 bis 900 °C getempert sind.

2. Kompositmaterial nach Anspruch 1 mit einem Füllstoff in einer Menge von 1 bis 90 Gew.-%.

3. Kompositmaterial nach Anspruch 1 oder 2, **dadurch gekennzeichnet, daß** der Füllstoff zusätzlich splitterförmige und/oder sphärische anorganische Füllstoffteilchen enthält.

4. Kompositmaterial nach Anspruch 1 bis 3, **dadurch gekennzeichnet, daß** der Füllstoff zusätzlich nicht torusförmige Füllstoffteilchen aus Siliziumdioxid enthält.

5. Kompositmaterial nach Anspruch 4, **dadurch gekennzeichnet, daß** die nicht torusförmigen Füllstoffteilchen aus pyrogener und/oder gefällter Kieselsäure und/oder aus Siliziumsolen und/oder aus einer Dispersion pyrogener und/oder aus gefällter Kieselsäure hergestellt sind.

6. Kompositmaterial nach einem der Ansprüche 1 bis 5, **dadurch gekennzeichnet, daß** die torusförmigen und/oder nicht torusförmigen Füllstoffteilchen silanisiert sind.

7. Kompositmaterial nach einem der Ansprüche 1 bis 6, **dadurch gekennzeichnet, daß** das organische Bindemittel mindestens einen der nachfolgenden Stoffe umfaßt: ethylenisch ungesättigte Monomere und Oligomere, Epoxide, Ormocere, Ceramere, flüssigkristalline Systeme, Spiroorthoester, Oxethane, Polyurethane, Polyester, A-Silikone und C-Silikone, Polycarbonsäuren.

8. Kompositmaterial nach einem der Ansprüche 1 bis 7, **dadurch gekennzeichnet, daß** das organische Bindemittel chemisch und/oder photochemisch härtet.

9. Kompositmaterial nach einem der Ansprüche 1 bis 8, **dadurch gekennzeichnet, daß** die torusförmigen Füllstoffteilchen einen mittleren Außendurchmesser im Bereich von 10 und 50 µm aufweisen.

10. Kompositmaterial nach einem der Ansprüche 1 bis 9, **dadurch gekennzeichnet, daß** die torusförmigen Füllstoffteilchen einen Innendurchmesser im Bereich von 0.2 - 20 µm aufweisen.

11. Kompositmaterial nach Anspruch 10, **dadurch gekennzeichnet, daß** die torusförmigen Füllstoffteilchen einen Innendurchmesser im Bereich von 0.4 - 4.0 µm aufweisen.

12. Kompositmaterial nach einem der Ansprüche 1 bis 11, **dadurch gekennzeichnet, dass** es 15 - 70 Gew.-% Füllstoff mit torusförmigen Füllstoffteilchen enthält.

13. Kompositmaterial nach einem der Ansprüche 1 bis 12, **dadurch gekennzeichnet, dass** die Füllstoffteilchen Siliziumdioxid und/oder Schwermetalloxide mit Ordnungszahlen größer 28 enthalten.

14. Kompositmaterial nach Anspruch 13, **dadurch gekennzeichnet, daß** die Schwermetalloxide aus der Gruppe der Zirkoniumoxide, Ceroxide, Zinnoxide, Zinkoxide, Yttriumoxide, Strontiumoxide, Bariumoxide, Lanthanoxide, Wismutoxide, sowie deren Mischungen gewählt werden.

15. Dentales Kompositmaterial nach einem der Ansprüche 1 bis 14.

16. Verwendung eines gefüllten und polymerisierbaren Kompositmaterials, das einen Füllstoff mit Füllstoffteilchen enthält, welche die Form eines Torus aufweisen, nach einem der Ansprüche 1 bis 15, als Dentalmaterial.

## Claims

1. Composite material with a polymerisable organic binder and a filler containing filler particles obtained by spray drying, which have the shape of a torus and an average external diameter in the region of 0.5-100 µm, **characterised in that** the filler particles with the shape of a torus are post-cured at temperatures in the range of 800 to 900 DEG C.

2. Composite material according to claim 1 with a filler in a quantity of 1 to 90 wt.%.

3. Composite material according to claim 1 or 2, **characterised in that** the filler contains additional fragment-shaped and/or spherical inorganic filler particles.

4. Composite material according to any of claims 1 to 3, **characterised in that** the filler additionally contains non-torus-shaped filler particles made from silicon dioxide.

5. Composite material according to claim 4, **characterised in that** the non-torus-shaped filler particles are produced from pyrogenic and/or precipitated silicic acid and/or silicon dioxide sols and/or from a dispersion of pyrogenic and/or precipitated silicic acid.

6. Composite material according to any of claims 1 to 5, **characterised in that** the torus-shaped and/or non-torus-shaped filler particles are silanized.

7. Composite material according to any of claims 1 to 6, **characterised in that** the organic binder includes at least one of the following materials: ethylenically unsaturated monomers and oligomers, epoxides, ormocers, ceramers, liquid crystal systems, spiro-orthoesters, oxethane, polyurethane, polyester, A-silicon and C-silicon, polycarbonic acids.

8. Composite material according to any of claims 1 to 7, **characterised in that** the organic binder cures chemically and/or photochemically.

9. Composite material according to any of claims 1 to 8, **characterised in that** the torus-shaped filler particles have an average external diameter in the region of 10 and 50 µm.

10. Composite material according to any of claims 1 to 9, **characterised in that** the torus-shaped filler particles have an internal diameter in the region of 0.2-20 µm.

11. Composite material according to claim 10, **characterised in that** the torus-shaped filler particles have an internal diameter in the region of 0.4-4.0 µm.

12. Composite material according to any of claims 1 to 11, **characterised in that** it contains 15-70 wt.% filler with torus-shaped filler particles.

13. Composite material according to any of claims 1 to 12, **characterised in that** the filler particles contain silicon dioxide and/or heavy metal oxides with an atomic number of greater than 28.

14. Composite material according to claim 13, **characterised in that** the heavy metal oxides are selected from the group of zirconium oxide, ceroxide, tin oxide, zinc oxide, yttrium oxide, strontium oxide, barium oxide, lanthanum oxide, bismuth oxide and compounds thereof.

15. Dental composite material according to any of claims 1 to 14.

16. Use of a filled and polymerisable composite material which contains a filler with filler particles which have the shape of a torus, in particular according to any of claims 1 to 15, as a dental material.

## Revendications

1. Matériau composite comprenant un liant organique polymérisable et une matière de remplissage qui contient des particules de matière de remplissage, qui présentent la forme d'un tore et un diamètre externe moyen dans l'intervalle de 0,5 à 100 µm, obtenues par séchage par pulvérisation de saumures, **caractérisé en ce que** les particules de matière de remplissage ayant la forme d'un tore sont recuites à une température dans l'intervalle de 800 à 900 °C.

2. Matériau composite selon la revendication 1, comprenant une matière de remplissage dans une quantité de 1 à 90 % en poids.

3. Matériau composite selon la revendication 1 ou 2, **caractérisé en ce que** la matière de remplissage contient en plus des particules de matière de remplissage anorganiques en forme d'éclats et/ou sphériques.

4. Matériau composite selon les revendications 1 à 3, **caractérisé en ce que** la matière de remplissage contient en plus des particules de matière de remplissage en silice qui ne sont pas en forme de tore.

5. Matériau composite selon la revendication 4, **caractérisé en ce que** les particules de matière de remplissage qui ne sont pas en forme de tore sont fabriquées à partir d'acide silicique pyrogène et/ou précipité et/ou de saumures de silicium et/ou d'une dispersion d'acide silicique pyrogène et/ou précipité.

6. Matériau composite selon l'une des revendications 1 à 5, **caractérisé en ce que** les particules de matière de remplissage en forme de tore et/ou pas en forme de tore sont silanisées.

7. Matériau composite selon l'une des revendications 1 à 6, **caractérisé en ce que** le liant organique comprend au moins l'une des substances suivantes : monomères et oligomères non saturés éthyléniquement, époxydes, ormocers, céramères, systèmes cristallins liquides, spiro-orthoesters, oxéthanes, polyuréthanes, polyesters, silicones A et silicones C, acides polycarboxyliques.

8. Matériau composite selon l'une des revendications 1 à 7, **caractérisé en ce que** le liant organique durcit de manière chimique et/ou photochimique.

9. Matériau composite selon l'une des revendications 1 à 8, **caractérisé en ce que** les particules de matière de remplissage en forme de tore présentent un diamètre externe moyen dans l'intervalle de 10 et 50 µm.

10. Matériau composite selon l'une des revendications 1 à 9, **caractérisé en ce que** les particules de matière de remplissage en forme de tore présentent un diamètre interne dans l'intervalle de 0,2 à 20 µm.

11. Matériau composite selon la revendication 10, **caractérisé en ce que** les particules de matière de remplissage en forme de tore présentent un diamètre interne dans l'intervalle de 0,4 à 4,0 µm.

12. Matériau composite selon l'une des revendications 1 à 11, **caractérisé en ce qu'**il contient 15 à 70 % en poids de matière de remplissage avec des particules de matière de remplissage en forme de tore.

13. Matériau composite selon l'une des revendications 1 à 12, **caractérisé en ce que** les particules de matière de remplissage contiennent de la silice et/ou des oxydes de métaux lourds avec des numéros atomiques supérieurs à 28.

14. Matériau composite selon la revendication 13, **caractérisé en ce que** les oxydes de métaux lourds sont choisis dans le groupe des oxydes de zirconium, oxydes de cérium, oxydes d'étain, oxydes de zinc, oxydes d'yttrium, oxydes de strontium, oxydes de baryum, oxydes de lanthane, oxydes de bismuth, ainsi que leurs mélanges.

15. Matériau composite dentaire selon l'une des revendications 1 à 14.

16. Utilisation d'un matériau composite rempli et polymérisable qui contient une matière de remplissage avec des particules de matière de remplissage qui présentent la forme d'un tore, selon l'une des revendications 1 à 15, en tant que matériau dentaire.
